# EUROPEAN PATENT SPECIFICATION

(11) **EP 0 724 878 B1**
(45) Date of publication and mention of the grant of the patent: **13.10.1999**
(21) Application number: 95303853.6
(22) Date of filing: 06.06.1995
(51) Int. Cl.: A61K 31/40

(54) **Use of melatonin for treating patients suffering from drug dependencies**
Verwendung von Melatonin zur Behandlung von Patienten die an Drogenabhängigkeit leiden
Utilisation de la mélatonine pour traiter les patients souffrant d'une dépendance vis à vis d'une drogue

(30) Priority: 01.02.1995 US 381535
(43) Date of publication of application: 07.08.1996
(73) Proprietor: NEURIM PHARMACEUTICALS (1991) LIMITED, Tel Aviv 69710 (IL)
(72) Inventor: Zisapel, Nava, Tel Aviv (IL)
(74) Representative: Hillier, Peter

(56) References cited:
- EP-A- 0 513 702
- EP-A- 0 518 468
- JOURNAL OF PINEAL RESEARCH, vol. 20, no. 2, 1996, pages 65-71, XP002004027 J. ATSMON ET AL.: "RECIPROCAL EFFECTS OF CHRONIC DIAZEPAM AND MELATOIN ON BRAIN MELATONIN AND BENZODIAZEPAPINE BINDING SITES"
- PHARMACOLOGY BIOCHEMISTRY AND BEHAVIOR, vol. 41, no. 2, 1992, pages 405-408, XP002004028 B. GUARDIOLA-LEMAITRE ET AL.: "COMBINED EFFECTS OF DIAZEPAM AND MELATONIN IN TWO TESTS FOR ANXIOLYTIC ACTIVITY IN THE MOUSE"
- JOURNAL OF PINEAL RESEARCH, vol. 15, no. 1, 1993, pages 1-12, XP002004029 D. DAWSON ET AL.: "MELATONIN AND SLEEP IN HUMANS"
- DIALOG FILE SUPPLIER: PHIND; AN=00302794; SCRIP 1700 P20, 13 March 1992, XP002004030 "POLIFARMA'S "SLEEP NORMALISER""
- CHEMICAL ABSTRACTS, vol. 121, no. 11, 12 September 1994 Columbus, Ohio, US; abstract no. 125040, A. HITOSHI ET AL.: "EFFECT OF INDOLPYRUVIC ACID ( A KETOANALOG OF TRYPTOPHAN) ON INDOLE METABOLISM IN THE BRAIN AND THE PINEAL GLAND" XP002004031

## Description

The present invention relates to melatonin for use in the manufacture of a medicament for treating, or for preventing, symptoms of dependence on, tolerance of, or addiction to benzodiazepine drugs, for treating multidrug addicts and to a pharmaceutical formulation, for use in such treatments

Dependence on benzodiazepines often develops in insomniacs who use them for the induction of sleep and in multi-drug addicts who in the process of withdrawal from narcotics, become addicted to benzodiazepines to ease anxiety and convulsions. Moreover, chronic benzodiazepine administration (where the benzodiazepines usually have long half-life values) may induce tolerance, expressed by an ineffective increase in dosage, by an unknown mechanism. Furthermore, rebound or "withdrawal" phenomena which often follow abrupt cessation of these drugs, as observed both in animals and humans lead to addiction (Greenblatt, D.J., and Shader, R.I., Drug Metab. Rev., 1978, 8: 13-28). In the 1990 US National Household Survey of the Use of Psychotherapeutic Medications, about 8% of the medical users of hypnotics advanced a prescribed dose on their own, which is an increase of 25% as compared to former report in 1979. Taking into consideration that the survey found that 2.6% of the US population took benzodiazepine hypnotics (as compared to 2.4% in 1979) the number of individuals in the US only who do develop tolerance and dependence may be estimated at 560,000. These values do not include substance use outside medical or social norms and multiple drug abuse. No method of rapid withdrawal followed by an effective alternative treatment has yet been reported in patients who developed dependence on benzodiazepine hypnotics and this problem is a great obstacle in the rehabilitation and recovery of narcotic drug addicts.

It is well known that melatonin, an indole-derived hormone produced at night by the pineal gland, plays a major role in mediating the circadian sleep-wake cycle and in the regulation of sleep. There is also some evidence that melatonin can increase benzodiazepine efficacy, see, e.g., Cardinali, D.P. et al, Adv. Biochem. Psychopharm., 1986, 42: 155- 164; Acuna Castroviejo, D., et al, J. Pineal Res., 1986, 3: 101-102; and Niles, L.P. et al, J. Neural Transm. 70: 117-124]. Also, melatonin can enhance the anxiolytic effects of diazepam in mice (Guardiola-Lemaitre, B. et al, Pharmacol. Biochem. Behav., 1992, 41, 405-4080). On the other hand, it has been suggested that benzodiazepines could, in some species including humans, potentiate GABA-induced inhibition of melatonin synthesis and secretion (McIntyre, I.M. et al, Biol. Psychiat., 1988, 24: 105-108) and that nocturnal enhancement of plasma melatonin could be suppressed by benzodiazepines in humans, thus leading to distortion in the diurnal melatonin rhythm (Kabuto, M. et al, Endocr. Japon., 1986, 33, 405-414). Moreover, it has been observed that chronic treatment with oxazepam modified the diurnal variations in the density of melatonin receptors at night in the rat brain and that this effect was not observed in pinealectomized animals (Anis, Y. et al, J. Neural Transm., 1992, 89: 155-166).

It has surprisingly been found in connection with the present invention that administration of melatonin concurrently with benzodiazepine drugs can potentially (1) wean a patient away from dependence on, addiction to, or tolerance of such drugs, and (2) in the case of a patient who has been diagnosed as requiring a benzodiazepine drug (where such undesired symptoms have not yet occurred), prevent the occurrence of such symptoms.

### ACKNOWLEDGMENT OF CITED PRIOR ART

EP-A-513702 describes the use of melatonin and certain derivatives thereof in the therapy of sleep disorders and in pre-anaesthetic medication, optionally in presence of a benzodiazepine. This document states that the benzodiazepine can thereby be administered in a relatively lower dosage, and thus avoid effects (alteration of sleep rhythms, rebound effect and development of tolerance) which are said to be related to administration of benzodiazepines in high dosages or for prolonged periods of time. This document does not suggest that dosages of melatonin or the derivatives less than 10 mg could be used for the stated purpose, neither does it suggest that melatonin might be useful when a patient has already become dependent on, tolerant of, or addicted to a benzodiazepine drug.

EP-A-518468 describes pharmaceutical controlled-release formulations which, briefly stated, release melatonin according to a profile which simulates the profile in plasma of a human having a normal endogenous melatonin profile. The formulations, which optionally contain a melatonin receptor profile modifier such as oxazepam, are useful for treating conditions related to melatonin deficiency or distortion, such as sudden infant death syndrome and migraine. This document does not suggest use of melatonin to prevent or treat addiction to benzodiazepines.

DIALOG File Supplier PHIND. AN 00302794,13-03-92, reports that indole-3-pyruvic acid (IPA) increases sleep time in insomniacs, e.g. in benzodiazepine withdrawal, and that the effect of IPA on sleep is mainly mediated by increased melatonin turnover in the pineal gland. This document does nor suggest use of melatonin to prevent or treat addiction to benzodiazepines.

### DESCRIPTION OF THE INVENTION

The present invention thus provides use of melatonin in the manufacture of a medicament for treating addiction to benzodiazepines in a multidrug addict, or a patient who has symptoms of having become dependent on, tolerant of, or addicted to a benzodiazepine drug, or for treating a patient who has been clinically diagnosed as having a condition susceptible to alleviation by administration of a benzodiazepine drug, while simultaneously preventing the occurrence in the patient of symptoms of dependence on, tolerance of, or addiction to said benzodiazepine drug, wherein said medicament contains at least an amount of melatonin effective for any of said treatments, said amount being adapted for a daily rate of administration within the range of 0.01-100 mg, provided that such use excludes use in the manufacture of a medicament containing 10-100 mg melatonin for treating a patient who has been clinically diagnosed as having a condition susceptible to alleviation by administration of a benzodiazepine drug, while simultaneously preventing the occurrence in the patient of symptoms of dependence on, tolerance of, or addiction to said benzodiazepine drug.

The said medicament may be a pharmaceutical formulation adapted for oral, rectal, parenteral or transdermal administration and which comprises at least one diluent, carrier or adjuvant, and may be additionally characterized by at least one of the following features: (i) it is in unit dosage form, each unit dosage comprising an amount of melatonin which lies within the range of 0.0025-100 mg; (ii) it is in the form of a controlled release formulation, wherein the melatonin is preferably released at a predetermined controlled rate; (iii) it comprises also at least one melatonin receptor modifier and/or melatonin profile modifier. The medicament may comprise also, and the pharmaceutical formulation according to the invention comprises, at least one benzodiazepine drug, such as at least one of Alprazolam, Chlordiazepoxide, Clorazepate, Diazepam, Flunitrazepam, Flurazepam, Halazepam, Lorazepam, Oxazepam, Prazepam, Temazepam and Triazolam. The formulation which comprises at least one benzodiazepine drug may also be characterized further by one or more of the features (i), (ii) and (iii) as described above.

In applying the present invention to treating a multidrug addict or a patient who has symptoms of having become dependent on, tolerant of, or addicted to a benzodiazepine drug, administration of a benzodiazepine drug to the patient is continued, at least initially, and melatonin is concurrently administered to the patient an amount which is effective to alleviate at least one of such symptoms.

In a particular embodiment of such treatment, either one of the benzodiazepine drug and the melatonin may be in the form of a pharmaceutical formulation adapted for oral, rectal, parenteral or transdermal administration and which comprises at least one diluent, carrier or adjuvant. Alternatively, benzodiazepine drug and melatonin may each be administered thus formulated, either separately, or may be combined into a single pharmaceutical formulation including both diazepine drug and melatonin.

In relation to the administration of the melatonin, whether administered separately from or together with one or more benzodiazepine drugs, administration may be effected at a daily dosage rate which e.g. lies within the range of 0.01-100 mg; it may be administered in the form of a controlled release formulation. Illustratively, 1-2 mg melatonin in the form of a controlled release formulation may be administered at night. The melatonin may be administered together with a melatonin receptor modifier or a melatonin profile modifier. Examples of melatonin receptor modifiers are short-acting benzodiazepines such as Oxazepam; examples of melatonin profile modifiers are benzodiazepines, beta-blockers and serotonin uptake inhibitors. Instead of, or in addition to, use of such a profile modifier, the melatonin profile may be modified by subjecting the patient to the effect of light, before, after or during administration of melatonin.

The benzodiazepine drugs referred to herein may give rise to symptoms of dependence, tolerance and/or addiction. Without prejudice to this generality, such drug or drugs may be one or more of, e.g., Alprazolam, Chlordiazepoxide, Clorazepate, Diazepam, Flunitrazepam, Flurazepam, Halazepam, Lorazepam, Oxazepam, Prazepam, Temazepam and Triazolam, as indicated above.

In one alternative embodiment of applying the invention to treating the above-mentioned symptoms, the benzodiazepine drug(s) is(are) initially continued to be administered to the patient, concurrently with the melatonin, at a daily rate substantially the same as that received by the patient prior to commencing treatment with melatonin. In another alternative embodiment of applying the invention in treating such symptoms, the benzodiazepine drug(s) is (are) administered to the patient, concurrently with the melatonin, at a progressively decreasing daily rate compared with that received by the patient prior to commencing treatment with melatonin. In this embodiment, the progressively decreasing daily rate of administration may be continued, e.g., until a predetermined stabilized rate of administration is achieved, or alternatively, e.g., until the amount of benzodiazepine drug administered is zero.

In applying the invention for preventive purposes, i.e. in treating a patient who has been clinically diagnosed as having a condition susceptible to alleviation by administration of a benzodiazepine drug, while simultaneously preventing the occurrence in the patient of symptoms of dependence on, tolerance of, or addiction to said benzodiazepine drug, a benzodiazepine drug is administered in an amount effective to alleviate said condition, while concurrently administering to the patient an amount of melatonin which is effective to prevent at least one of such symptoms. The various embodiments described above as applicable to treating a patient having the stated symptom(s) are also correspondingly applicable to preventive purposes, except insofar as they will not be applicable for reasons which are self-evident to a person of the art, e.g. in this instance treatment with a benzodiazepine drug is a desideratum, so that evidently the amount of benzodiazepine administered, while possibly being reduced in any particular case as determined by a physician, will not be reduced to zero.

However, it will be within the scope of the preventive application of the invention, not only to administer, concurrently with melatonin, the benzodiazepine drug(s) at the conventional daily dosage rate to achieve a particular purpose, but in the alternative to similarly administer such drug(s) at a daily rate which is less than that which is conventionally administered to a patient in order to alleviate said condition.

As stated above, the invention also extends to a pharmaceutical formulation which includes at least one a benzodiazepine drug and melatonin. Since benzodiazepine drugs are usually administered 1-4 times daily, a daily rate of 0.01-100 mg melatonin, administered typically at night, in the same formulation as the benzodiazepine(s), or even if administered separately therefrom, will illustratively be achieved by administering benzodiazepines as follows:

| days | unit dosage of benzodiazepines within the range |
|---|---|
| 1 | 0.01-100 mg |
| 2 | 0.05- 50 mg |
| 3 | 0.033-33.3 mg |
| 4 | 0.025-25 mg |

Thus, when the pharmaceutical formulation of the invention is in unit dosage form, each unit dosage is preferably administered at night and preferably comprises an amount of melatonin within the range 0.0025-100 mg.

The following Table gives the amounts of benzodiazepine drugs used for treating the stated conditions in adults. For further information, e.g. as to reservations, half-life, forms of administration and suitable dosages for children or infants, see Goodman & Gilman's 'The Pharmacological Basis of Therapeutics" 7th Edition, 1985 (MacMillan Publishing Co.), the passages relating to use of benzodiazepines (e.g. pp. 352, 437).

| Benzodiazepine | Content of unit oral dosage mg (x per day) | | Usual daily oral dose * |
|---|---|---|---|
| | Sedative | Hypnotic | Anxiolytic |
| Alprazolam | | | 0.75-1.5 |
| Chlordiazepoxide | 10-100(1-3) | 50-100 | 15-40 |
| Clorazepate | 3.75-15(2-4) | 15-30 | 30 |
| Diazepam | 5-10(3-4) | 5-10 | 4-40 |
| Flurazepam | | 15-30 | |
| Halazepam | | | 60-160 |
| Lorazepam | | 2- 4 | 2- 6 |
| Oxazepam | 15-30(3-4) | 15-30 | 30-60 |
| Prazepam | | | 20-40 |
| Temazepam | | 15-30 | |
| Triazolam | | 0.25-0.5 | |

| | | | |
|---|---|---|---|
| *mg; generally divided into 2-4 unit doses; for further information including parenteral dosage rates, see Goodman & Gilman, *loc cit* | | | |

The preparation and release profile of formulations for use in accordance with the invention or its applications are illustrated below.
(a) There were compressed in a 7 mm cylindrical punch at 2540 kg (2.5 tons), after dry mixing of the powdered materials, namely 2 mg/tablet melatonin (Biosynth Co., Switzerland) and acrylic resin carrier (Rohm Pharma) which was Eudragit® RS100 (formulation SR-Ms) or Eudragit® RSPO (formulation SR-Mf), besides other components as noted: formulation SR-Ms: Eudragit® RS100 48.8%, lactose 50%, melatonin 1.2%; formulation SR-Mf: Eudragit® RSPO 35.3%, lactose 16.7%, calcium hydrogen phosphate 41.4%, talc 1.3%, magnesium stearate 4%, melatonin 1.3%. SR-Ms and SR-Mf are sustained release formulations.
A conventional dosage form (RM) was prepared similarly to formulation SR-Mf, but using lactose instead of Eudragit® as carrier.
(b) The potential release profile of the tablets prepared as described in paragraph (a), was first investigated by in vitro dissolution of melatonin therefrom in distilled water at 37°C. The results in Table A show the % of the melatonin content (mean value of 6 tablets) which has dissolved at the stated intervals of time.

**Table A**

| Time(hours) | 1 | 2 | 4 | 6 | 8 | 10 |
|---|---|---|---|---|---|---|
| melatonin (%) released from: | | | | | | |
| SR-Ms | 12 | 29 | 62 | 84 | 90 | 100 |
| SR-Mf | 32 | 51 | 76 | 88 | 100 | |
| RM | 93 | 96 | 100 | | | |

(c) The in vivo profile of the SR-Mf tablets prepared as described in paragraph (a), was investigated by oral administration twice to a healthy male (age 36) at 10 a.m., i.e. when circulating melatonin levels are undetectable. The amount of melatonin released in vivo was determined by radioimmunoassay of its major metabolite, 6-sulphatoxymelatonin, in the urine. The amount of urinary 6-sulphatoxymelatonin closely reflects the blood level of the hormone. The results in Table B show the melatonin determined as a % of the total melatonin administered (mean value of 2 tablets).

**Table B**

| In vivo release of melatonin from SR-Mf | | | | | | |
|---|---|---|---|---|---|---|
| Time(hours) | 1 | 2 | 4 | 6 | 8 | 10 |
| % release at intervals | 10.7 | 25.7 | 40.6 | 14.0 | 7.0 | 1.9 |
| cumulative release % | 10.7 | 36.4 | 77.0 | 91.0 | 98.0 | 99.9 |

It is noted that the release of melatonin in vitro, illustrated in Table A, provides only an approximate indication of the in vivo release profile due to the known phenomenon of the active compound being absorbed by the tissues in the early stages of release.

The amount of melatonin in the sustained release formulations may be changed e.g. to 0.5, 1 or 5 mg/tablet, without affecting the release pattern found for the tablets containing 2 mg/tablet melatonin.

Insofar as analogues of melatonin which substantially imitate the function of melatonin in the human body are known in the art, it will be appreciated that such analogues are deemed to be obvious chemical equivalents of melatonin, in the present context.

In accordance with the present invention, one or more benzodiazepines may be incorporated in the above formulations, in amounts which have been described herein.

The invention will now be illustrated by the following Examples.

### EXAMPLE 1

The reciprocal effects of chronic benzodiazepine and melatonin administration on brain melatonin and benzodiazepine receptors and the ability of melatonin to reverse these effects were studied. Male rats were maintained on a daily 14 h light:10 h darkness schedule (lights-on 05.00h; cool white fluorescent illumination) at 24±2°C. Food and drinking water were supplied ad libitum. The animals (2 months old), were divided into 4 groups, 5 animals in each. The animals in one group (CON) were injected i.p. daily at 16:00 h with vehicle (200 µl saline). Those in the second group (VAL) were injected daily, i.p. at 16:00 h with diazepam (1 mg in 200 µl vehicle; Roche). The animals of the third group (MEL) were injected daily at 16:00 h with vehicle; the drinking water of this group contained melatonin (4 mg dissolved in 100 µl ethanol and diluted to 1 liter). The animals in the fourth group (VAL/MEL) were injected daily at 16:00 h with diazepam (1 mg in 200 µl vehicle); the drinking water of this group contained melatonin (4 mg dissolved in 100 µl ethanol and diluted to 1 liter). After 21 days the treatment was stopped and the animals were weighed. The mean body weight values in the VAL (274±20 g) and VAL/MEL groups (239±30 g) were found to be slightly lower than those in CON (292±30 g) or MEL groups (285±30 g).

The animals were decapitated between 18-19.00 h of the next day (at this time the density of 2-¹²⁵I-iodomelatonin in the medulla pons should be maximal); their brains were rapidly removed and crude synaptosomal pellets were prepared as described, and melatonin receptors were assessed, as described by Laudon, M. and Zisapel, N., FEBS Lett., 1986, 197: 9-12. Benzodiazepine receptors were assessed by measuring ³H-flunitrazepam (³H-FNZ) and ³H-RO 15-1788 binding as described by Amiri, Z. et al, Brain Res., 1991, 553: 155-158. Binding parameters were calculated from the equilibrium binding data. Bmax values represent the specific binding of 2-¹²⁵I-iodomelatonin, ³H-FNZ or ³H-RO 15-1788 at saturation, and Kd values are the apparent dissociation constants. Binding parameters of the various groups were compared by analysis of Variance followed by Student-Newman-Keul's test for multiple comparisons. Differences were considered significant if P<0.05. Daily injections of diazepam (1 mg i.p at 16.00 h) to male rats for 3 weeks markedly reduced the density of 2-¹²⁵I-iodomelatonin binding sites in the medulla-pons (Table 1), whereas benzodiazepine binding was not significantly affected (Table 2). If melatonin receptors are related to the control of the sleep-wake cycle, the results suggest that chronic benzodiazepine administration results in the diminution of melatonin-responsive mechanisms and consequent physiological activities.

Melatonin, given orally via the drinking water for 3 weeks, significantly enhanced ³H-RO 15-1788 binding in the medulla pons (Tables 2,3), whereas 2-¹²⁵I-iodomelatonin binding was not affected. The increase in benzodiazepine binding site density and apparent Kd in the medulla-pons induced by the melatonin treatment, is compatible with the augmentation observed previously in the rat cortex, and shown to be mediated by opioid peptides (Gomar, M.D. et al, Neuroendocrinology 1993, 4:987-990). The fact that this enhancement persists even in diazepam-treated animals may rule out competition between melatonin and benzodiazepines on the benzodiazepine binding sites.

Daily administration of both diazepam and melatonin, enhanced ³H-RO 15-1788 binding in the medulla pons, and reversed the diazepam-induced suppression of 2-¹²⁵I-iodomelatonin binding in this area (Tables 1,2). These results are surprising since as previously shown (Anis, Y. et al, in melatonin binding sites in the hamster brain: impact of melatonin. Molec. Cell. Endocrinol., 1989, 67: 121-128; Oaknin-Bendahan, S. et al, J. Basic Clin. Physiol. Pharmacol., 1992, 3: 253-268) and is also confirmed in the present study, administration of melatonin by morning or evening injections, or orally via the drinking water, does not affect the density or diurnal variations in melatonin binding sites in most brain areas including medulla-pons. Moreover, pinealectomy does not abolish the diurnal variations in 2-¹²⁵I-iodomelatonin binding sites although it affects their phase position (Oaknin-Bendahan et al., 1992, ibid). Thus, changes in melatonin binding site densities might not be due to autoregulation of the receptor by melatonin.

In the cerebral cortex, melatonin slightly reduced ³H-RO 15-1788 and ³H-FNZ binding. Diazepam treatment did not significantly affect ³H-RO 15-1788 and ³H-FNZ binding but prevented the melatonin-mediated decrease (Tables 2,3). These data suggest firstly, that the effects of melatonin on benzodiazepine binding sites are localized, rather than general suppression or facilitation of the binding occurring, and secondly, that melatonin replacement therapy may counteract some deleterious effects of chronic benzodiazepine treatment.

Table 1 shows equilibrium binding parameters of 2-¹²⁵I-iodomelatonin binding sites in synaptosomal preparations from the medulla-pons area of diazepam and/or melatonin-treated and untreated rats, in terms of mean and S.D. values of Kd (in nM) and Bmax (in µmol/mg protein). Values denoted by the same character In Table 1 do not differ significantly. (Codes having the same significance are also used in Tables 2 and 3, below.)

**Table 1**

| GROUP | Kd±sd | Bmax±sd |
|---|---|---|
| CON | 0.87±0.2 a | 7.9±1.0 a |
| MEL | 1.16±0.3 a | 7.7±1.0 a |
| VAL | 0.98±0.21 a | 5.1±0.5 b |
| VAL/MEL | 2.27±0.75 b | 12.5±2.0 c |

Table 2 shows equilibrium binding parameters of ³H-RO 15-1788 binding sites in synaptosomal preparations from the medulla-pons area of diazepam and/or melatonin-treated and untreated rats, in terms of mean and S.D. values of Kd (in nM) and Bmax (in µmol/mg protein).

**Table 2**

| GROUP | Kd±sd | Bmax±sd |
|---|---|---|
| CON | 2.3±0.4 a | 310±22 a |
| MEL | 2.8±0.2 a | 476±26 b |
| VAL | 2.5±0.4 a | 295±34 a |
| VAL/MEL | 2.6±0.5 a | 375±87 b |

Table 3 shows the effect of diazepam or melatonin on ³H-FNZ and ³H-RO 15-1788 binding in rat cerebral cortex membranes, in terms of mean and S.D. values (in µmol/mg protein).

**Table 3**

| GROUP | ³H-FNZ | ³H-RO 15-1788 |
|---|---|---|
| CON | 935±31 a | 1354± 48 a |
| MEL | 765±78 b | 1060± 26 b |
| VAL | 870±22 a | 1264± 99 a |
| VAL/MEL | 980±16 a | 1362±155 a |

### EXAMPLE 2

This Example illustrates the surprising action of melatonin in facilitating very rapid withdrawal from benzodiazepine drug tolerance. A 43 year old female, married with 2 children has been suffering from sleep onset insomnia for the last 10 years accompanied by frequent and severe migraine attacks. A thorough neurological assessment was negative. Psychiatric or other organic problems were also ruled out. Throughout these years she had been treated with benzodiazepines, tricyclic antidepressants and neuroleptic drugs, as well as by biofeedback and relaxation methods, with no apparent relief. For the last year she has been using 4-8 mg Lorazepam every night.

A thorough psychological assessment at the Sleep Laboratory of Tel Aviv University, did not disclose any significant pathology. The quality of sleep was assessed by an actigraph tracing which automatically monitors the bedtime sleep-wake pattern through a small device attached to the hand wrist. The tracing was recorded for 3 consecutive days and showed a deranged sleep pattern: reduced efficiency, long sleep latency and multiple waking episodes. Urine was collected every 3 hours (for 36 hours) and assayed for the major melatonin metabolite: 6-sulphatoxymelatonin, as an indicator for the diurnal secretion of plasma melatonin. Results showed that 6-sulphatoxymelatonin excretion levels were lower than for aged-matched individuals, and lacked the typical circadian rhythm (TABLE 4).

Oral administration of a controlled-release melatonin formulation in the form of tablets containing 1 mg melatonin (Neurim Pharmaceuticals, Israel) was initiated, in order to correct for the deficiency and distortion of the melatonin rhythm. One tablet was administered daily at 8:30 pm. The patient was asked to gradually reduce the number of benzodiazepine tablets taken each night. Surprisingly, within 2 days, the patient stopped using the benzodiazepine hypnotics altogether, and claimed that her insomnia has improved remarkably. In addition, her headaches also subsided gradually. A repeated actigraph tracing after 3 weeks treatment showed marked improvement in sleep pattern.

The treatment was stopped and 2 weeks afterwards urine was collected again every 3 hours (for 36 hours) and assayed for 6-sulphatoxymelatonin. The results (Table 4) indicated an increase in amount and a clear nocturnal peak of urinary 6-sulphatoxymelatonin. A 5-month follow-up has confirmed that the patient still maintains her quality of sleep and hardly suffers from headaches. After 6 months without treatment sleep quality began to deteriorate and melatonin therapy was resumed.

This case-report indicates potentially a breakthrough in relieving many patients whose quality of life has been impaired by addiction to benzodiazepine hypnotics. Administration of exogenous melatonin may moreover serve as a means of rapid and symptomless withdrawal from benzodiazepines in tolerant patients.

**Table 4**

| Urinary 6-sulphatoxymelatonin in benzodiazepine-dependent patient before and after melatonin therapy (µg/hour) | | |
|---|---|---|
| Time | before treatment | after treatment |
| 15.00 | 0.3 | 0.11 |
| 18.00 | 0.16 | 0.45 |
| 21.00 | 0.18 | 0.11 |
| 24.00 | 0.13 | 1.24 |
| 3.00 | 0.23 | 0.74 |
| 6.00 | 0.23 | 0.36 |
| 9.00 | 0.22 | 0.21 |
| 12.00 | 0.13 | 0.01 |
| 15.00 | 0.22 | 0.04 |

### EXAMPLE 3

This example illustrates the effects of long term administration of melatonin in the treatment of insomnia in patients dependent on a benzodiazepine drug.

Two volunteers, Y.L., an 80 year old male, and E.L., a 73 year old female, had each suffered for a number of years from insomnia and/or frequent awakenings during the night accompanied by difficulty in resuming sleep afterwards. Both were found to have low melatonin secretion, by determination of the amount of the metabolite 6-sulphatoxymelatonin, in the urine. Both patients had been taking 1-2 mg of flunitrazepam orally prior to retiring each evening.

Each patient was weaned off the flunitrazepam by gradually reducing the dose and simultaneously administering melatonin orally (2 mg melatonin daily in controlled release form) over a two-month period. Since the end of that period, each patient has continued taking melatonin in the same form and at the same dosage rate over approximately two years.

Each patient has subjectively reported good sleep inducement and a substantial improvement in sleep quality. Specifically, patient E.L. noted an improvement in sleep quality at the beginning of the weaning period and Y.L. noted a similar effect about two weeks into the weaning period. Each patient reported reduced fatigue during the daytime within several days after the beginning of the weaning period, and also indicated that the melatonin has caused neither residual tiredness in the morning, nor any hangover feeling. No side effects were reported by either patient.

### EXAMPLE 4

This example, designed as a randomized, double-blind, crossover study, illustrates the ability of melatonin replacement therapy to improve sleep maintenance in chronic benzodiazepine drug-using elderly patients.

The group, of mean age 78 (SD=9.7) consisted of eight men and five women, all of whom complained of long-term insomnia and used various benzodiazepines for sleep induction. Urine was collected approximately every 4 hours for 15 hours and the nocturnal excretion of 6-sulphatoxymelatonin, the major urinary metabolite of melatonin, was assayed in duplicate by RIA. Urine analysis of these patients showed low and delayed 6-sulphatoxymelatonin excretion (<14 µg per night compared with 25 µg per minute in young adults). The study protocol consisted of two treatment periods of three weeks each, with one week wash-out interval between the two treatment periods. During the treatment periods, patients were administered orally either 2 mg controlled-release melatonin tablets, or placebo, two hours before bedtime. Five patients continued the melatonin treatment for a period of two months beyond the initial experimental period.

Patients' sleep was objectively assessed at the end of each treatment period for three consecutive nights using a wrist-worn actigraph. Motion recordings were analysed using the Neurim algorithm to determine sleep latency, sleep efficiency, total slepp time, wake after sleep onset and number of awakenings, as an average over three nights for each subject. Six Wilcoxon matched-pairs signed-ranks analyses revealed statistically significant differences in sleep parameters between the melatonin and placebo treatment periods' ranks. The results are shown in Table 5.

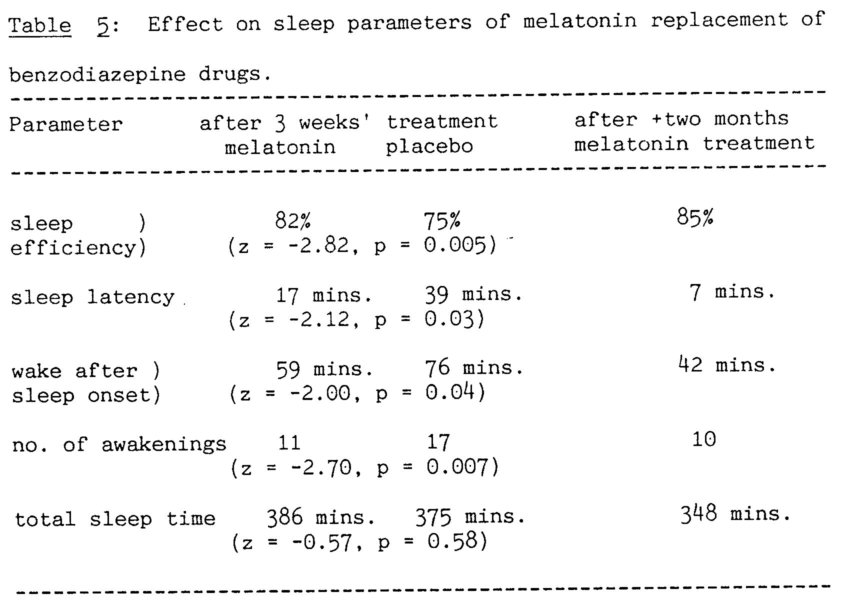

From the above results, it is concluded that melatonin replacement therapy can improve sleep initiation and maintenance in benzodiazepine drug-using elderly patients having a low endogenous melatonin output. The benefits of melatonin treatment increase with time, suggesting that reorganisation of the circadian system has occurred.

## Claims

1. Use of melatonin in the manufacture of a medicament for treating addiction to benzodiazepines in a multidrug addict, or a patient who has symptoms of having become dependent on, tolerant of, or addicted to a benzodiazepine drug, or for treating a patient who has been clinically diagnosed as having a condition susceptible to alleviation by administration of a benzodiazepine drug, while simultaneously preventing the occurrence in the patient of symptoms of dependence on, tolerance of, or addiction to said benzodiazepine drug, wherein said medicament contains at least an amount of melatonin effective for any of said treatments, said amount being adapted for a daily rate of administration within the range of 0.01-100 mg, provided that such use excludes use in the manufacture of a medicament containing 10-100 mg melatonin for treating a patient who has been clinically diagnosed as having a condition susceptible to alleviation by administration of a benzodiazepine drug, while simultaneously preventing the occurrence in the patient of symptoms of dependence on, tolerance of, or addiction to said benzodiazepine drug.

2. Use of melatonin according to claim 1 in the manufacture of a medicament for treating addiction to benzodiazepines in a multidrug addict, or a patient who has symptoms of having become dependent on, tolerant of or addicted to a benzodiazepine drug.

3. Use of melatonin according to claim 1 in the manufacture of a controlled release medicament for treating a patient who has been clinically diagnosed as having a condition susceptible to alleviation by administration of a benzodiazepine drug, while simultaneously preventing the occurrence in the patient of symptoms of dependence on, tolerance of, or addiction to said benzodiazepine drug.

4. Use according to any one of claims 1-3, wherein said medicament comprises a pharmaceutical formulation adapted for oral, rectal, parenteral or transdermal administration and which comprises at least one diluent, carrier or adjuvant.

5. Use according to claim 4, wherein said pharmaceutical formulation is additionally characterized by at least one of the following features:
(i) it is in unit dosage form, each unit dosage comprising an amount of melatonin which lies within the range of 0.0025-100 mg;
(ii) it is a controlled release formulation, wherein the melatonin is released at a predetermined controlled rate;
(iii) it comprises also at least one melatonin receptor modifier and/or melatonin profile modifier.

6. Use according to any one of claims 1-5, wherein said pharmaceutical formulation comprises also at least one benzodiazepine drug, which preferably comprises at least one of Alprazolam, Chlordiazepoxide, Clorazepate, Diazepam, Flunitrazepam, Flurazepam, Halazepam, Lorazepam, Oxazepam, Prazepam, Temazepam and Triazolam.

7. Use according to any one of claims 1-6, wherein said medicament manufactured by use of melatonin is to be administered concurrently with the benzodiazepine drug(s), the latter to be administered at a daily rate which is progressively decreased until a predetermined stabilized rate of administration of benzodiazepine drug(s) is achieved.

8. Use according to any one of claims 1-6, wherein said medicament manufactured by use of melatonin is to be administered concurrently with the benzodiazepine drug(s), the latter to be administered at a daily rate which is progressively decreased until complete withdrawal of the patient from benzodiazepine drug(s) is achieved.

9. Use according to any one of claims 1-8, wherein said medicament is a sustained release tablet, which contains at least 0.0025 mg and no more than 5 mg melatonin.

## Patentansprüche

1. Verwendung von Melatonin bei der Herstellung eines Medikaments zur Behandlung Von Sucht nach Benzodiazepinen bei einem Multi-Drogensüchtigen oder einem Patienten, der Symptome aufweist, abhängig von, tolerant gegen oder süchtig nach einem Benzodiazepin-Arzneimittel geworden zu sein, oder zur Behandlung eines Patienten, von dem klinisch die Diagnose gestellt wurde, ein Leiden zu haben, das für Linderung durch Verabreichung eines Benzodiazepin-Arzneimittels empfänglich ist, während gleichzeitig das Auftreten von Symptomen der Abhängigkeit von, der Toleranz gegen oder der Sucht nach dem Benzodiazepin-Arzneimittel bei dem Patienten verhindert wird, wobei das Medikament mindestens eine Menge an Melatonin enthält, die für eine der Behandlungen wirksam ist, wobei die Menge an eine tägliche Menge der Verabreichung in dem Bereich von 0,01-100 mg angepaßt ist, mit der Maßgabe, daß eine derartige Verwendung ausschließt die Verwendung bei der Herstellung eines Medikaments, das 10-100 mg Melatonin enthält, zur Behandlung eines Patienten, von dem klinisch die Diagnose gestellt wurde. ein Leiden zu haben, das für Linderung durch Verabreichung eines Benzodiazepin-Arzneimittels empfänglich ist, während gleichzeitig das Auftreten von Symptomen der Abhängigkeit von, der Toleranz gegen oder der Sucht nach dem Benzodiazepin-Arzneimittel bei dem Patienten verhindert wird.

2. Verwendung von Melatonin gemäß Anspruch 1 bei der Herstellung eines Medikaments zur Behandlung von Sucht nach Benzodiazepinen bei einem Multi-Drogensüchtigen oder einem Patienten, der Symptome aufweist, abhängig von, tolerant gegen oder süchtig nach einem Benzodiazepin-Arzneimittel geworden zu sein.

3. Verwendung von Melatoningemäß Anspruch 1 bei der Herstellung eines Medikaments mit geregelter Freisetzung zur Behandlung eines Patienten, von dem klinisch die Diagnose gestellt wurde, ein Leiden zu haben, das für Linderung durch Verabreichung eines Benzodiazepin Arzneimittels empfänglich ist, während gleichzeitig das Auftreten von Symptomen der Abhängigkeit von, der Toleranz gegen oder der Sucht flach dem Benzodiazepin-Arzneimittel bei dem Patienten verhindert wird.

4. Verwendung gemäß einem der Ansprüche 1-3, wobei das Medikament eine pharmazeutische Zubereitung umfaßt, die an orale, rektale, parenterale oder transdermale Verabreichung angepaßt ist und die mindestens ein Verdünnungsmittel, Träger oder Adjuvans umfaßt.

5. Verwendung gemäß Anspruch 4, wobei die pharmazeutische Zubereitung zusätzlich gekennzeichnet ist durch mindestens eines der folgenden Merkmale:
(i) sie liegt in einheitlicher Dosierungsform vor, wobei jede einheitliche Dosierung eine Menge an Melatonin umfaßt, die in dem Bereich von 0,0025-100 mg liegt;
(ii) sie ist eine Zubereitung mit geregelter Freisetzung, wobei das Melatonin in einer vorherbestimmten geregelten Menge freigesetzt wird;
(iii) sie umfaßt außerdem mindestens ein Melatonin-Rezeptor-Modifizierungsmittel und/oder Melatonin-Profil-Modifizierungsmittel.

6. Verwendung gemäß einem der Ansprüche 1-5, wobei die pharmazeutische Zubereitung außerdem mindestens ein Benzodiazepin-Arzneimittel umfaßt, das vorzugsweise mindestens eines von Alprazolam, Chlordiazepoxid, Clorazepat, Diazepam, Flunitrazepam, Flurazepam, Halazepam, Lorazepam, Oxazepam. Prazepam, Temazepam und Triazolam umfaßt.

7. Verwendung gemäß einem der Ansprüche 1-6, wobei das durch die Verwendung von Melatonin hergestellte Medikament gleichzeitig mit dem (den) Benzodiazepin-Arzneimittel(n) zu verabreichen ist, wobei das letztere in einer täglichen Menge zu verabreichen ist, die zunehmend vermindert wird. bis eine vorherbestimmte stabilisierte Menge der Verabreichung von Benzodiazepin-Arzneimittel(n) erreicht ist.

8. Verwendung gemäß einem der Ansprüche 1-6, wobei das durch die Verwendung von Melatonin hergestellte Medikament gleichzeitig mit dem (den) Benzodiazepin-Arzneimittel(n) zu verabreichen ist, wobei das letztere in einer täglichen Menge zu verabreichen ist, die zunehmend vermindert wird, bis ein vollständiger Entzug des Patienten von dem (den) Benzodiazepin-Arzneimittel(n) erreicht ist.

9. Verwendung gemäß einem der Ansprüche 1-8, wobei das Medikament eine Depot-Tablette ist, die mindestens 0,0025 mg und nicht mehr als 5 mg Melatonin enthält.

## Revendications

1. Utilisation de mélatonine dans la fabrication d'un médicament pour traiter l'addiction aux benzodiazépines chez un polytoxicomane, ou un patient qui présente des symptômes de dépendance, de tolérance ou d'addiction à un médicament à benzodiazépine, ou pour traiter un patient chez lequel il est diagnostiqué cliniquement une condition susceptible d'être soulagée par l'administration d'un médicament à benzodiazépine, tout en prévenant simultanément la survenue chez ce patient de symptômes de dépendance, de tolérance ou d'addiction audit médicament à benzodiazépine, ledit médicament contenant au moins une quantité de mélatonine efficace pour l'un quelconque desdits traitements, ladite quantité étant adaptée pour une dose quotidienne d'administration dans la gamme de 0,01 à 100 mg, étant entendu qu'une telle utilisation exclut l'utilisation dans la fabrication d'un médicament contenant 10 à 100 mg de mélatonine pour traiter un patient chez lequel il a été diagnostiqué cliniquement une condition susceptible d'être soulagée par l'administration d'un médicament à benzodiazépine, tout en prévenant simultanément la survenue chez ce patient de symptômes de dépendance, de tolérance ou d'addiction audit médicament à benzodiazépine.

2. Utilisation de mélatonine selon la revendication 1, dans la fabrication d'un médicament pour traiter l'addiction aux benzodiazépines chez un polytoxicomane, ou un patient qui présente des symptômes de dépendance, de tolérance ou d'addiction à un médicament à benzodiazépine.

3. Utilisation de mélatonine selon la revendication 1, dans la fabrication d'un médicament à libération contrôlée pour traiter un patient chez lequel il a été diagnostiqué cliniquement une condition susceptible d'être soulagée par l'administration d'un médicament à benzodiazépine,tout en prévenant simultanément la survenue chez ce patient de symptômes de dépendance, de tolérance ou d'addiction audit médicament à benzodiazépine.

4. Utilisation selon l'une quelconque des revendications 1-3, dans laquelle ledit médicament comprend une formulation pharmaceutique adaptée pour l'administration orale, rectale, parentérale ou transdermique et qui comprend au moins un diluant, véhicule ou adjuvant.

5. Utilisation selon la revendication 4, dans laquelle ladite formulation pharmaceutique est de plus caractérisée par au moins l'une des caractéristiques suivantes:
(i) elle est sous forme de dosage unitaire, chaque dosage unitaire comprenant une quantité de mélatonine qui se trouve dans la gamme de 0,0025 à 100 mg;
(ii) c'est une formulation à libération contrôlée, dans laquelle la mélatonine est libérée à une vitesse contrôlée prédéterminée:
(iii) elle comprend aussi au moins un modificateur des récepteurs de mélatonine et/ou un modificateur du profil de la mélatonine.

6. Utilisation selon l'une quelconque des revendications 1-5, dans laquelle ladite formulation pharmaceutique comprend aussi au moins un médicament à benzodiazépine, qui comprend de préférence au moins l'un parmi l'Alprazolam, le Chlordiazépoxyde, le Clorazépate, le Diazépam, le Flunitrazépam, le Flurazépam, le Halazépam, le Lorazépam, l'Oxazépam, le Prazépam, le Témazépam et le Triazolam.

7. Utilisation selon l'une quelconque des revendications 1-6, dans laquelle ledit médicament manufacturé en utilisant de la mélatonine doit être administré en même temps que le(s) médicament(s) à benzodiazépine, ce dernier devant être administré à une dose quotidienne qui est progressivement diminuée jusqu'à ce qu'une dose d'administration du (des) médicament(s) à benzodiazépine stabilisée prédéterminée soit atteinte.

8. Utilisation selon l'une quelconque des revendications 1-6, dans laquelle ledit médicament fabriqué en utilisant de la mélatonine doit être administré en même temps que le(s) médicament(s) à benzodiazépine, ce dernier devant être administré à une dose quotidienne qui est progressivement diminuée jusqu'à ce que le sevrage complet du patient du (des) médicament(s) à benzodiazépine soit atteint.

9. Utilisation selon l'une quelconque des revendications 1-8, dans laquelle ledit médicament est un comprimé à libération prolongée, qui contient au moins 0,0025 mg et pas plus de 5 mg de mélatonine.
